# EUROPEAN PATENT SPECIFICATION

(11) **EP 1 735 327 B1**
(45) Date of publication and mention of the grant of the patent: **04.05.2011**
(21) Application number: 04770659.3
(22) Date of filing: 20.05.2004
(51) Int. Cl.: C07H 1/06, C07H 5/02

(54) **AN IMPROVED PROCESS FOR PRODUCING CHLORINATED SUCROSE**
VERBESSERTES VERFAHREN ZUR HERSTELLUNG VON CHLORIERTER SACCHAROSE
PROCEDE AMELIORE POUR LA PRODUCTION DE SACCHAROSE CHLORE

(30) Priority: 19.03.2004 WO PCT/IN2004/000064; 17.05.2004 IN MU05632004
(43) Date of publication of application: 27.12.2006
(73) Proprietor: V.B. MEDICARE PVT. LTD., Bangalore 560 048 KRN (IN)
(72) Inventor: RATNAM, Rakesh c/o Pharmed Medicare Private Ltd., Mumbai, Maharashtra, 400 001 (IN); KULKARNI, Shrikant c/o Pharmed Medicare Priv. Ltd., Mumbai, Maharashtra, 400 001 (IN); AURORA, Suneet c/o Pharmed Medicare Private Ltd., Mumbai, Maharashtra, 400 001 (IN)
(74) Representative: Kuhnen & Wacker
(86) International application number: PCT/IN2004/000142
(87) International publication number: WO 2005/090374

(56) References cited:
- US-A- 4 380 476
- US-A- 5 498 709
- FAIRCLOUGH P H ET AL: "DERIVATIVES OF BETA-D-FRUCTOFURANOSYL ALPHA-D-GALACTO-PYRANOSIDE" CARBOHYDRATE RESEARCH, AMSTERDAM, NL, vol. 40, no. 2, 1 April 1975 (1975-04-01), pages 285-298, XP000571304

## Description

### TECHNICAL FIELD

This invention relates to an improved process for producing chlorinated sucrose

### BACKGROUND OF THE INVENTION

Chloro derivatives derived from sugars, exhibit the organoleptic properties with a very high degree of sweetness compared to the parent sugar. One such chloro sugar prepared from sucrose is 1', 6'-Dichloro-1', 6'-Dideoxy-β-D-Fructo-Furanosyl-4-Chloro-4-Deoxy-α-D-Galactopyranoside. It is a well-known sweetener used widely, including in food and food preparations. Various synthetic routes for the production of 1', 6'-Dichloro-1', 6'-Dideoxy-β-D-Fructo-Furanosyl-4-Chloro-4-Deoxy-α-D-Galactopyranoside are reported in literature, for e.g. Fairclough et al, Carbohydrate Research 40(1975) 285-298; Mufti et.al. 1983 US Patent No. 4,380,476; Walkup, et al. 1990 US Patent no. 4,980,463 and British Patent No. 1543167. They involve preparing derivatives of sucrose, chlorinating them and to recover the desired product, chlorinated sucrose, by reversing derivatization. Preferred processes involve converting sucrose into acetate, chlorination of the acetate, deacylation of the chlorinated acetate and recovering the product from reaction mixture.

A major challenge in these approaches is to separate the desired product, 1', 6'-Dichloro-1', 6'-Dideoxy-β-D-Fructo-Furanosyl-4-Chloro-4-Deoxy-α-D-Galactopyranoside from the chlorinated mass, from other chloro derivatives of sucrose, salts formed during reaction, dark degradation products and tars formed on account of oxidation and elimination due to the relatively severe conditions of chlorination reaction, and finally all these solid components from the large volume of liquids consisting of solvents such as tertiary amide.

Processes for production of this compound reported in patent literature are all based on handling these liquid reaction mixtures by subjecting them, in liquid state itself, to selective liquid-liquid extraction by using organic solvents, column chromatographic purification of the solvent extracts, and the intermediate derivatives and or the chlorinated sucrose is recovered by conventional crystallization procedure. The procedures used so far for removal of tertiary amides from these liquid reaction mixtures as well as conventional crystallization are very elaborate, cumbersome to operate and control and expensive on account of the cost of equipment needed and energy required to run the processes.

Navia et al, 1996 US Patent No. 5498709 describes a process for preparing chlorinated sucrose where removal of tertiary amide from the liquid reaction mixture is done by steam distillation. However, the solvent being a high boiling point, it takes a very long time for it to be stripped off to the maximum limits. Also it is reported in the same patent that the volumes of the mass increases to 4 to 5 times of the original volume. This increase in volume also increases the time for isolation of the product as well as increases size of the processing plant to that extent for handling the resultant volume of reactants for further processing.

An improved process, having several potential economic advantages, for preparation of chlorinated sucrose is disclosed in co-pending application No: PCT/IN04/0064. This improved process described recovery of solids, from liquids containing chlorinated sucrose or its intermediates with or without other solids, obtained in a process for producing chlorinated sucrose or otherwise, mainly 1', 6'-Dichloro-1', 6'-Dideoxy-β-D-Fructo-Furanosyl-4-Chloro-4-Deoxy-α-D-Galactopyranoside by using drying, under mild controlled conditions by industrially used processes, as a more efficient and more effective tool. The described drying process, is applicable to any liquid solution obtained as reaction mixture or purified solution in course of any process including in the course of process for production of chlorinated sucrose or its intermediates. The drying methods which are subject of this co-pending application include, but are not limited to, agitated thin film drying (ATFD), spray drying, freeze drying and super critical extraction. The preferred drying process used in co-pending application was ATFD.

The drying method, as applied in the preceding para has been very effectively used in improving efficiency of operation of post-chlorination steps and recovery of final product intermediate (acetate) and final product in pure solid form. Drying of reaction mixture after completion of chlorination reaction achieved total removal of tertiary amides from the system in a most convenient and most effective way in very short time and better control is obtained on volumes to be handled and effective application of post chlorination steps such as deacylation. Drying also affords a most convenient and more effective and more efficient alternative to batch or continuous processes (some of which described by Navia, et al., U.S. Pat. No. 5,498,706; Mufti, et al., U.S. Pat. No. 4,380,476) of crystallization based on conventional cumbersome crystallization procedures used so far for recovery of final product intermediate or final product from purified liquids obtained in course of production process. The final powder of the product or product intermediate obtained by drying process is amorphous in nature and not crystalline as in conventional crystallization procedure, both having same organoleptic taste and chemical analysis. However, the amorphous powder obtained in the invented process shall have different physical properties such as free-flowing powder properties, different storage properties than the crystalline variety. In that sense, amorphous powder is a new product and its properties are being studied.

### SUMMARY OF INVENTION

In present invention as claimed in appended claim 1, which is in continuation with the invention disclosed in co-pending application no. PCT/IN04/0064, it was found that efficiency improvement achieved by improvements claimed in the above mentioned co-pending application improves further and becomes more flexible by introducing following alternative routes of further process:
i) Adjusting the pH of the reaction mixture after chlorination to a variety of chosen alkaline ranges, well below pH 11, either neutralization to pH 7.0 to 7.5, or adjusting to pH 7.5 to 9.
ii) Deacylation being done either before or after drying the reaction mixture by drying methods including agitated thin film drying (ATFD).
iii) Use of alkoxides in the steps for deacylation.

These steps have been very effectively combined with mild methods of drying claimed in PCT/IN04/0064application of the applicants of this patent application to construct very efficient alternative routes for production of chlorinated sucrose having high potential of economic advantages. Some of the alternative routes constructed are as follows:
1. Drying directly the reaction mixture of chlorination step being undertaken of the whole reaction mixture solution either by direct feeding at pH 7.0 to 7.5, or after adjustment of pH to 7.5 to 9.0 or after deacylation step.
2. Drying directly the wash liquid collected from chromatography or any separation method resulting into a purified solution of chlorinated sucrose or its intermediate or derivative for recovery of the product or the product derivative in solid powder form;
3. Dissolving the ATFD dried solids of the chlorination reaction mixture in water, extracting the same in appropriate organic solvent, and drying the solvent extract by ATFD to recover the product or product intermediate in solid powder form;
4. Concentrating the solvent extract obtained in any step of production of chlorinated sucrose, and drying the concentrated extracts to recover the product or product intermediate in solid powder form; or
5. Drying the solution of chlorinated sugar, its derivatives or intermediates obtained otherwise than in the process of production of chlorinated sugars.

The detailed description and the specific examples given in a subsequent section, for the purpose of indicating specific embodiments of the invention, serve the purpose of illustration only. Accordingly, the present invention includes also those various changes and modifications which come within the spirit and scope of the invention that may become obvious to those skilled in the art from this detailed description.

### BRIEF DESCRIPTION OF DRAWINGS AND SHORTFORMS

POCL3 (POCl₃) stands for Phosphorous oxy-chloride
ATFD stands for Agitated Thin Film Dryer
TLC stands for Thin Layer Chromatography
HPLC stands for High Pressure Liquid Chromatography

Fig. 1 illustrates the reaction scheme for the preparation of 1', 6'-Dichloro-1', 6'-Dideoxy-β-D-Fructo-Furanosyl-4-Chloro-4-Deoxy-α-D-Galactopyranoside.

Fig. 2 illustrates the agitated thin film dryer used in the process of the present invention,

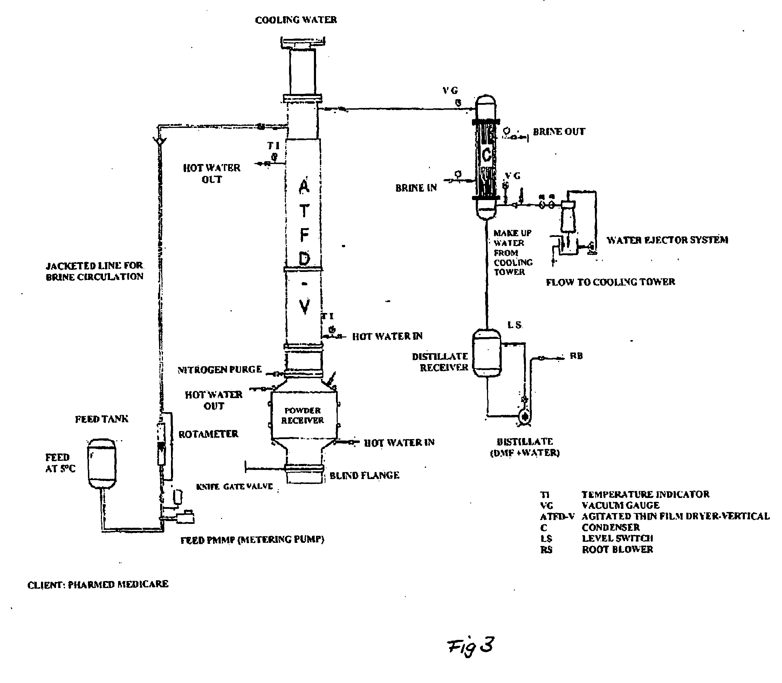

Fig. 3 is the flow sheet of the agitated thin film dryer;

Fig. 4 is the flow chart of the process of the present invention,

Fig. 5 is the IR Report of the product of the present invention; and

Fig. 6 is the HPLC Chromatogram of the product of the present invention.

### DETAILED DESCRIPTION OF THE INVENTION

It is understood that the customary rules of Interpretation of patent documents apply to this document also. However, some of those interpretations, which will expressly apply, are mentioned below.

Present invention is not limited to the particular methodologies, protocols, solvents, and reagents, etc, described herein as tools to achieve the claimed objective, as these may vary with change in context. Further, the terminology used herein is used for the purpose of describing particular embodiments only, and is not intended to and shall not limit the scope of the present invention.

As used herein and in the appended claims, the singular forms "a" "an" and " the" include plural reference also unless the context clearly indicates or requires or means otherwise. Thus, for example, a reference to "a process" is a reference to one or more processes for the stipulated objective and includes equivalents thereof known or obvious to those skilled in the art and so forth. Unless defined otherwise or contrary to the context, all technical and scientific terms used herein have the same meanings as commonly understood by one of ordinary skill in the art to which this invention belongs. Several methods, devices, and materials are described herein, although in practice or for testing of present invention, any methods and materials similar or equivalent to those described herein can be used. All documents cited herein are to be construed for reference and interpretation herein in their entirety

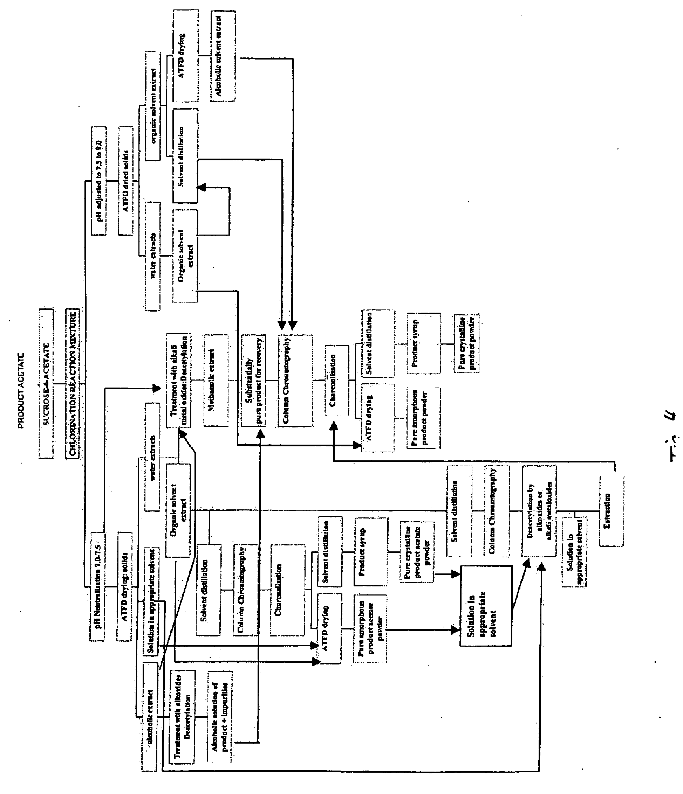

The process of drying under mild conditions applied to any liquid containing chlorinated sucrose derivative, either in substantially pure form or with other accompanying solid ingredients, is illustrated in Fig 4. Strategic use of some important modifications and the advantages of these modifications and subjecting the liquid solutions subjected to direct drying under mild conditions and certain other modifications introduced in the process, in the context of process efficiency, are as follows:
1. The reaction mixture of chlorination step, after completion of the reaction, can be subjected to drying under mild conditions either after or before deacylation step. This brings in significant improvement in process efficiencies, as it is more easy to handle a dry powder for further purification of the chlorinated sucrose or its intermediate in absence of solvent than in presence of solvent. It is pertinent to mention that the final product as well as inorganic salts formed during the process are both highly soluble in water. Hence, to extract the desired product completely from aqueous medium may be a difficult proposition. However, the extraction of solids (comprising of desired product or its intermediate and inorganic salts) by alcoholic organic solvent is better solution as selective extraction of the desired product will be more easy. The drying process requires less equipment, less consumption of energy, offers freedom from tediousness of crystallization processes in a batch or continuous operation. Several alternative procedures are available to adopt further course of operation either by extracting the chlorinated sucrose or its intermediate either by using a suitable organic solvent, ethyl acetate being preferable, or by supercritical extraction, or by dissolving the solids in water and subjecting the same to purification by column chromatography. Removal of the solvents by drying in above step is more convenient than by steam stripping adopted by Navia et al., in U.S. Pat. No. 5,498,709.
2. Further, once substantially pure solution of chlorinated sucrose or its intermediates or derivatives is obtained in the course of process of their production, recovery of the product is very convenient, direct, total and most effective by resorting to drying under mild conditions than by recovering desired product by conventional crystallization from their concentrated solutions of those products as such or of their derivatives. The process of drying gives substantially pure product directly in one step process in short time with least problems of control without any residual losses of solids; their purity, chemical properties and functional properties such as taste are same as the product obtained by conventional crystallization. This is a decided advantage in view of the cumbersomeness and expenses involved in conventional crystallization and in any process based on conventional crystallization.
3. Such substantially pure solutions of chlorinated sucrose or their intermediates, which could be submitted to drying under mild conditions for recovery of their solids for specific processing advantages, are obtained in the process illustrated in Fig 4 in the form of wash liquid coming out of chromatographic column when extracts obtained from solids of drying of chlorination reaction mixture are chromatographed and such wash liquid is evaporated and dissolved in appropriate volume of methanol.

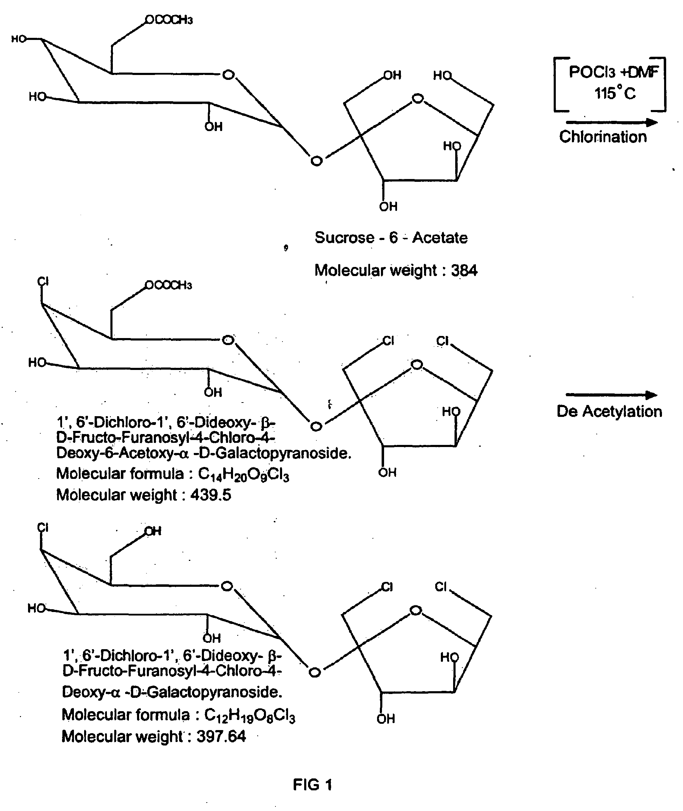

In the methods preferred here for the illustration of this invention:
1. The chlorinated sucrose is 1',6'-Dichloro-1',6'-Dideoxy-β-D-Fructo-Furanosyl-4-Chloro-4-Deoxy-α-D-Galactopyranoside and also its acetate intermediates.
2. Sucrose-6-ester is sucrose-6-acetate.
3. The method of drying under mild conditions is Agitated Thin Film Drying, which involves evaporation achieved by using a vertical agitated thin film evaporator with hinged rotor blades.
4. The reaction scheme in general involved in the manufacture of product from sucrose-6- acetate is given in fig. 1 of the accompanying drawings. Many permutations and combinations could be done in the indicated scheme, some of which, but not including all, are shown as illustration in the fig 1.

Various operations of the process of production of 1', 6'-Dichloro-1', 6'-, Dideoxy-β-D-Fructo-Furanosyl-4-Chloro-4-Deoxy-α-D-Galactopyranoside are as follows:
The sucrose-6-acetate is chlorinated by the Vilsemeier reagent, which is prepared from Phosphorus Oxy Chloride (POCl3) or phosphorus penta chloride (PCl5). The sucrose-6-acetate is added to Vilsmeier Reagent at 5.to 10. °C. After completion of the reaction, the reaction mass is heated to 80.. to 100. °C. and preferably between 90. to 95. °C. and maintained for ½ -1hr and then the temperature is raised to 110. to 135. °C. and preferably to 120. to125. °C. and maintained for 3-5 hours.

Thereafter the reaction mass is cooled to room temperature and neutralized using alkali hydroxide or carbonate solution.

The desired product 1', 6'-Dichloro-1',6'-Dideoxy-α-D-Fructo-Furanosyl-4-Chloro-4-Deoxy-α-D-Galactopyranoside or its acetate, obtained after or before deacylation respectively, could be isolated by using the appropriate method mentioned in Methods 1 to 5 given as follows:
**Method 1**: When the reaction mass after chlorination is directly fed into ATFD, the product obtained was a solid mixture of 1', 6'-Dichloro-1', 6'-Dideoxy-β-D-Fructo-Furanosyl-4-Chloro-4-Deoxy-6-acetoxy-α-D-Galactopyranoside-6-acetate and inorganic salts. The solids were treated with alcoholic solvents and filtered off to remove the inorganics. The alcoholic solution was then heated with appropriate organic alkoxides like sodium methoxide, sodium ethoxide, propoxides or its potassium analogs, during which the deacylation occurs to afford the desired product. The product could be isolated as a solid by either subjecting the alcoholic solution to the ATFD or the alcoholic solution could be further purified by column chromatography and the liquid eluted from the chromatographic column could be subjected to crystallization or direct drying by subjecting to ATFD.
**Method 2**: The solid mixture of 1', 6'-Dichloro-1', 6'-Dideoxy-β-D-Fructo-Furanosyl-4-Chloro-4-Deoxy-6-acetoxy-α-D-Galactopyranoside-6-acetate and inorganic salts from the reaction mixture could be dissolved in water or the reaction solution can be further treated directly without ATFD drying with the alkali metal oxides like sodium hydroxide or potassium hydroxide or even with alkali earth metals like calcium hydroxide or barium hydroxide, etc. The resultant solution could be fed into the ATFD or spray drier to remove water and afford solids, where now essentially will be comprising 1',6'-Dichloro-1', 6'-Dideoxy-β-D-Fructo-Furanosyl-4-Chloro-4-Deoxy-α-D-Galactopyranoside (the product) and alkali or alkaline earth metal chlorides (NaCl, KCI or CaCl₂). The product can be separated from inorganic chlorides by extracting the solids by organic solvents like methanol, ethyl acetate, acetone, etc and the product in the solvent extract can be further purified by column chromatography followed by crystallization or drying in ATFD.
**Method 3**: Yet another appropriate process was followed by adjusting the reaction mass to pH 7.5- 9.0 and then subjecting the reaction mass to ATFD for solvent stripping. The product obtained being the mixture of desired deacylate of 1', 6'-Dichloro-1', 6'-Dideoxy-β-D-Fructo-Furanosyl-4-Chloro-4-Deoxy-6-acetoxy-α-D-Galactopyranoside-6-acetate and inorganic salts. Effectively the deacylation was achieved in situ during the solvent stripping operations of ATFD. The solids obtained were treated with organic solvents to remove inorganic salts. The crude product after solvent stripping either by spray drying or ATFD was further purified after extracting solids in alcoholic solvents by column chromatography followed by crystallization or subjecting the chromatography column eluate to ATFD drying.
**Method 4**: isolation of the pure 1', 6'-Dichloro-1', 6'-Dideoxy-β-D-Fructo-Furanosyl-4-Chloro-4-Deoxy-6-acetoxy-α-D-Galactopyranoside-6-acetate from solids containing 1', 6'-Dichloro-1', 6'-Dideoxy-β-D-Fructo-Furanosyl-4-Chloro-4-Deoxy-6-acetoxy-α-D-Galactopyranoside-6-acetate and inorganic salts in a process mentioned in methods 1 and 2 could also be achieved by direct column chromatography of the salts obtained from ATFD or spray drier.
**Method 5**: isolation of the pure 1', 6'-Dichloro-1', 6'-Dideoxy-β-D-Fructo-Furanosyl-4-Chloro-4-Deoxy-6-acetoxy-α-D-Galactopyranoside-6-acetate from solids containing 1', 6'-Dichloro-1', 6'-Dideoxy-β-D-Fructo-Furanosyl-4-Chloro-4-Deoxy-6-acetoxy-α-D-Galactopyranoside-6-acetate and inorganic salts could also be achieved by dissolving the solids with water and extracting with organic solvents like dichloromethane, ethyl acetate and then stripping of solvent by drying by ATFD to get crude solid mixture. Isolation of the pure 1', 6'-Dichloro-1', 6'-Dideoxy-β-D-Fructo-Furanosyl-4-Chloro-4-Deoxy-6-acetoxy-α-D-Galactopyranoside from solids containing 1', 6'-Dichloro-1', 6'-Dideoxy-β-D-Fructo-Furanosyl-4-Chloro-4-Deoxy-6-acetoxy-α-D-Galactopyranoside-6-acetate can be achieved after followed by deacylation as mentioned in method1 and 2.
**Method 6**: isolation of the product 1', 6'-Dichloro-1', 6'-Dideoxy-β-D-Fructo-Furanosyl-4-Chloro-4-Deoxy-α-D-Galactopyranoside from solids containing 1', 6'-Dichloro-1', 6'-Dideoxy-β-D-Fructo-Furanosyl-4-Chloro-4-Deoxy-α-D-Galactopyranoside and inorganic salts could be done by dissolving the solids obtained by using process described in Method 3 in water and extracting the product in organic solvent like ethyl acetate, dichloromethane, etc. This was followed by solvent stripping, column chromatography and crystallization.

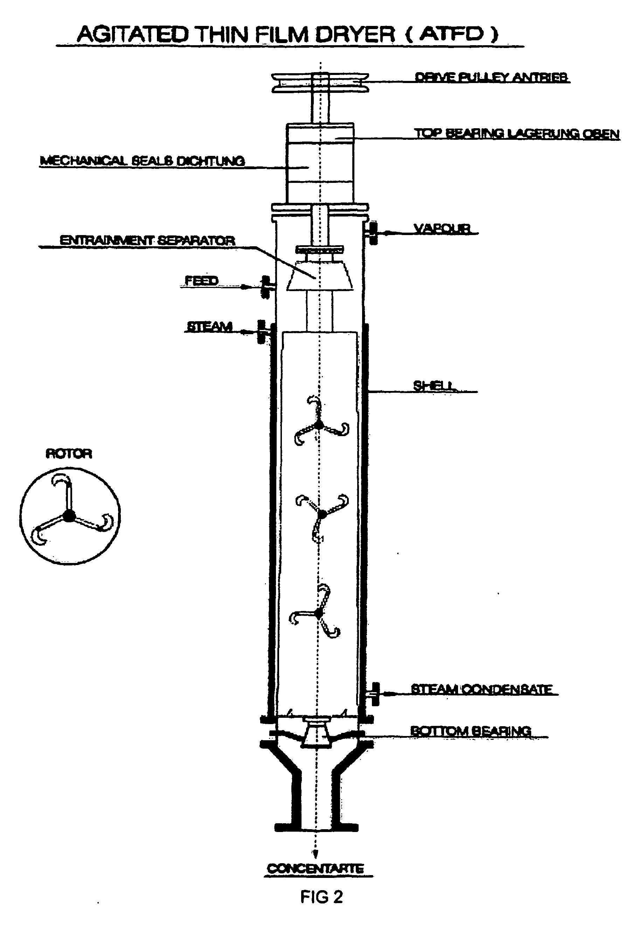

Diagram of ATFD is shown in Fig. 2 of the drawings.

The detailed preferred process of ATFD is illustrated as follows:
Feed of the reaction mass was cooled to 5. to 10. °C. in the feed tank by circulating a brine solution. A pump was used to lift the feed from feed tank to the Dryer. The ATFD is a vertical Dryer with area of cross section 0.25 to 0.35 m². The feed enters tangentially and spreads along the inside surface of the shell in to a thin film. The rotor blades are hinged; the hinged rotor blades keep the film under intense agitation preventing any scale formation. The speed of the rotor was 1000 to 1500 revolutions per minute. The film progressively passes through different phases like liquid, slurry, paste, wet powder and finely powder of desired dryness, it is collected in a powder receiver.

The vapor flows countercurrent to the solids and was removed from the top of the Dryer. Distillate was collected from the condenser and solids are obtained from the Dryer. The distillate contains solvent and water. The distillate was subjected to fractional distillation, about 70-80% of solvent was recovered based on the input of solvent.

### EXPERIMENT

### 1. CHLORINATION OF SUCROSE-6-ACETATE

100 g of sucrose-6-acetate was mixed with 200 ml of fresh solvent such as hexane, cyclohexane, pyridine, dimethyl formamide, and others, and particularly dimethyl formamide and Chlorination undertaken in a 3 liter 3 neck round bottom flask. 500 ml of the solvent was charged. Thereafter, the solvent was cooled with stirring to 0. to 5. °C. To this reaction mass, 166 ml of phosphorous oxy chloride (273.9 g) was added below 0. °C. To this chlorinating reagent, 100grm of sucrose 6-acetate in solvent was added below 10. °C. Thereafter, the reaction mass was stirred at 20. to 25. °C. for ½-1 hr. Further, the temperature was raised to 70..to 100. °C. and preferably 80. to 90. °C. and was maintained for 1 to 2 hr. Afterwards the temperature was raised to 110. to 130. °C. and preferably 120. to 122. °C. and was maintained for 3 to 5 hr. The reaction mass was cooled to 40. to 45. °C. and was neutralized.

### 2. ATFD REMOVAL OF SOLVENT

The reaction mass obtained after completion of chlorination reaction, which approximates volume of 2-2.3 liter, was further treated in one of the following, including but not limited to, two alternatives:
a) pH is adjusted to 7.0-7.5 to obtain mixture of 1', 6'-Dichloro-1', 6'-Dideoxy-β-D-Fructo-Furanosyl-4-Chloro-4-Deoxy-6-acetoxy-α-D-Galactopyranoside-6-acetate and inorganic salts;
b) pH is adjusted to 7.5 -9.0 to obtain mixture of 6-acetyl intermediate of 1', 6'-Dichloro-1', 6'-Dideoxy-β-D-Fructo-Furanosyl-4-Chloro-4-Deoxy-α-D-Galactopyranoside and inorganic salts;
and the reaction mass was then fed to ATFD with the parameters, which are described, but not limited to, as follows:.
Area of ATFD = 0.20 - 0.50 m²
Feed rate= 7-10 kg/hr
Pressure =2-10 mm Hg.
Jacket temp = 70. to 100.°C.

Feed of 3 to 5 kg was cooled down to 5..to 10. °C. in the feed tank by circulating the brine solution. pH of the feed was maintained at 7.0 to 7.5 or 7.5 to 9; the pump was fitted to lift the feed from feed tank to the dryer. The dryer is a vertical dryer with area of cross section 0.25-0.35 square Meter. The feed entered tangentially and spreads along the inside surface of the shell in to a thin film. The rotor blades are hinged, the hinged rotor blades keep the film under intense agitation preventing any scale formation. The speed of the rotor was 1000-1500 revolutions per minute. Temperature was maintained around 70..to 100. °C. in the jacket by circulating hot water taking inlet from bottom, outlet through the top. The film progressively passed through different phases like liquid, slurry, paste, wet powder and finely powder of desired dryness. This was collected in a powder receiver.

The dry product, which was essentially, a mixture of 1', 6'-Dichloro-1', 6'-Dideoxy-β-D-Fructo-Furanosyl-4-Chloro-4-Deoxy-6-acetoxy-α-D-Galactopyranoside-6-acetate and inorganic salts or alternatively deacylated 1', 6'-Dichloro-1', 6'-Dideoxy-α-D-Fructo-Furanosyl-4-Chloro-4-Deoxy-6-acetoxy-α-D-Galactopyranoside-6-acetate and inorganic salts. This depends on the respective feed to the ATFD as described above.

The vapor flows countercurrent to the solids and was removed from the top of the Dryer. These vapors were condensed in the condenser. Distillate was collected from the condenser, solids were obtained from the Dryer. The distillate contains solvent and water.

The distillate was subjected to fractional distillation, about 70-80% of solvent was recovered based on the input of solvent.

### 3. RECOVERY OF 1', 6'-DICHLORO-1', 6'-DIDEOXY-β-D-FRUCTO-FURANOSYL-4-CHLORO-4-DEOXY-α-D-GALACTOPYRANOSIDE FROM 6-ACETYL INTERMEDIATE, ITS DEACYLATION AND RECOVERY OF FINAL PRODUCT:

This was achieved in four alternative ways exemplified as follows:
3.1 The pH of the reaction mass after chlorination was adjusted to 7.0 - 7.5. The reaction mass was subjected to ATFD to obtain 450g of mixture of 1', 6'-Dichloro-1', 6'-Dideoxy-β-D-Fructo-Furanosyl-4-Chloro-4-Deoxy-6-acetoxy-α-D-Galactopyranoside-6-acetate and other inorganic salts. This powder was dissolved in 1.2 liters of water and complete dissolution was ensured. The solution was then extracted with 1 liter of ethyl acetate and layers separated. The aqueous solution was re-extracted with 400ml of ethyl acetate three times and all the ethyl acetate layers were pooled. The ethyl acetate was distilled off and the residue obtained was purified by column chromatography on silica gel. The purified 6-acetyl intermediate was deacylated with 10% sodium methoxide solution in methanol (pH 9 -10).
   The deacylated product was concentrated and crystallized to obtain pure 1', 6'-Dichloro-1', 6'-Dideoxy-β-D-Fructo-Furanosyl-4-Chloro-4-Deoxy-α-D-Galactopyranoside of 98.6% purity by HPLC and yield 30%.
3.2 450 g solids obtained from the ATFD which contains the 6-acetyl intermediate of 1', 6'-Dichloro-1', 6'-Dideoxy-β-D-Fructo-Furanosyl-4-Chloro-4-Deoxy-α-D-Galactopyranoside and the inorganic salts were dissolved in 500ml methanol three times. The methanolic extract was filtered to remove the inorganic salts.
   The methanolic solution containing 6-acetyl product was treated with sodium methoxide at room temperature and was mixed till complete deacetylation. The product was then concentrated to thick syrup and was taken for silica gel column chromatography. The purified fractions were collected, concentrated and crystallized to get pure product of 98.9% and yield 34%.
3.3 The pH of the chlorinated mass was adjusted to 8.5 - 9.0 and was fed to the ATFD. Solids obtained from ATFD, 450g, were extracted into 500 ml methanol three times. The methanol extract was filtered and concentrated and the residue was purified by silica gel column chromatography. The product obtained was 92% pure.
3.4 Solids from ATFD of the step of drying in example 3, 450g, were dissolved in 1.2 liter water and then extracted into 1:1 ethyl acetate. The aqueous solution was further extracted with 400 ml of ethyl acetate thrice. The ethyl acetate layers were pooled together and concentrated. The thick syrup obtained was purified by column chromatography to yield pure product of 93%.

### 4. CHROMATOGRAPHIC PURIFICATION AND CHARCOALIZATION

Purification of liquids containing product or product intermediates with impurities was purified by column chromatograpgy on silica gel or alumina using appropriate elution / desorbtion medium. The eluent coming out of the column was given charcoal bed treatment and was then sent for either ATFD drying or for conventional crystallization.

### 5. EXTRACTION OF PRODUCT FROM SOLIDS OBTAINED AFTER ATFD DRYING OF PURIFIED LIQUIDS CONTAING THE PRODUCT

The solid mass obtained from the ATFD, when the liquid subjected to drying contained almost pure product, was subjected to solvent extraction. The solvent used may be any organic solvent, including but not limited to, ethyl acetate, methanol, methyl ethyl ketone, and acetone. The preferred solvent used was methanol.

The solvent extracted mass was distilled in the rotary evaporator at low temperature. The syrup obtained was mixed with an appropriate column chromatography adsorbent like silica gel or alumina and run through column chromatography. The adsorbing agent could be any known column packing preferably alumina or silica gel. The preferable solvents for desorbption are ethyl acetate, mixture of toluene and methanol, mixture of methanol and ethyl acetate, mixture of methanol and dichloromethane. Ethyl Acetate was used in this work. The eluted fractions were collected in different receivers based on TLC showings. The fractions showing single spot on the TLC were collected separately. The solvent from this fraction was evaporated to provide a thick syrup. The thick syrup was subjected to purification. Crude product obtained showed by TLC to have a high concentration of the desired product. This was subjected to crystallization.

The products that are extracted and purified by the above processes were and could be any one of the following i.e. 1', 6'-Dichloro-1 6'-Dideoxy-p-D-Fructo-Furanosyl-4-Chloro-4-Deoxy-6-acetoxy-α-D-Galactopyranoside-6-acetate or its deacylated form i.e. 1',6'-Dichloro-1', 6'-Dideoxy-α-D-Fructo-Furanosyl-4-Chloro-4-Deoxy-6-acetoxy-α-D-Galactopyranoside.

### 6. ISOLATION OF PRODUCT ACETATE OR THE PRODUCT IN POWDER FORM

The syrup obtained from the isolation stage was mixed with 3-5 volumes of mass of ethyl acetate and mixed well. The mass was distilled at low temperature to remove 2-4 volumes of ethyl acetate resulting in a liquid product concentrate. The desired dry product was obtained from the liquid concentrate by three methods,
a) Conventional crystallization, as reported as in the earlier patents cited in this document. Essential steps consisted of solvent distillation to form a product syrup, which was then dissolved in minimum quantity of appropriate solvent and then seeded with the product crystals for crystallization. The pure Crystals were recovered by centrifugation.
b) Feeding liquid product concentrate to ATFD to obtain the dry desired pure product of the acetyl intermediate or the desired deacylated product. This has been reported for the first time in the co-pending application of these applicants No. PCT/N04/D064.
c) Spray drying the liquid product concentrate to obtain of the acetyl intermediate or the desired deacylated product, which has also been reported for the first time in the co-pending application of these applicants No. PCT/N04/D064.

In the conventional crystallization method the liquid concentrate was crystallized to obtain solid product. The product was filtered and dried under vacuum at 40. to 50. °C.

### 7. PROPERTIES OF THE PRODUCT OBTAINED THROUGH ABOVE EXPERIMENTS

The solids isolated after drying the purified product containing liquids in the ATFD were also found to be identical in taste, organoleptically, and chemical analysis with the product obtained from the conventional crystallization method. Also the solids obtained after spray drying the liquid concentrate were found to be identical in taste and chemical purity with the desired pure product obtained from the crystallization and ATFD method. Solid powders obtained by ATFD and other methods of drying when compared to powders obtained from crystallization procedures were, however, amorphous in nature having smaller particle size.

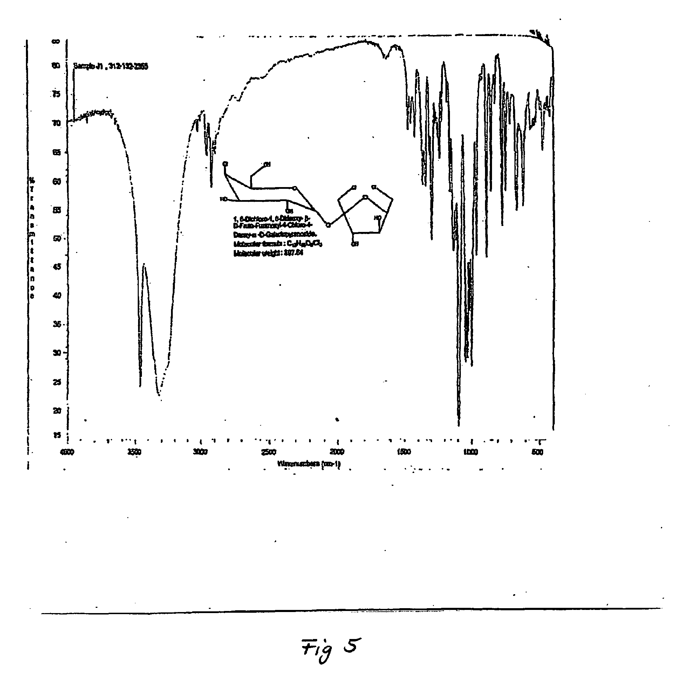

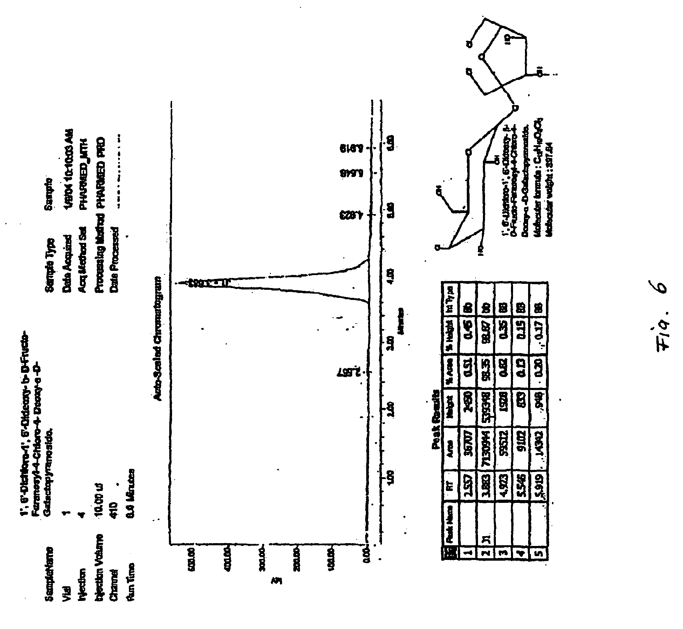

The chemical analysis of the solids from all the three methods showed the product purity or content was over 99% (HPLC Fig. 6 and IR Fig. 5 attached). In a separate experiment, the ethyl acetate extract from after column chromatography was directly passed through ATFD. This also resulted in a desired pure product of high purity.

The solid product could be also isolated by feeding the ethyl acetate extract after column chromatography directly into the Spray Dryer or any other Dryer to afford the solid product.

## Claims

1. A process of handling solution of sucrose intermediates and derivatives, including, chlorinated sucrose, comprising:
a) removal of liquids from the said solution by direct drying, under conditions mild enough to prevent degradation or modification of chlorinated sucrose, for recovery of solids from the said liquids and the end product of such operations is a solid mass of the chemicals visibly free from the said liquid;
b) recovering the said solids, present in the said liquid either in substantially pure form or with other solid impurities;
c) the said liquids being obtained in a process of producing chlorinated sucrose, mainly 1', 6'-Dichloro-1', 6'-Dideoxy-β-D-Fructo-Furanosyl-4-Chloro-4-Deoxy-α-D-Galactopyranoside;
the said method of drying including one or a combination of, agitated thin film drying, spray drying, freeze drying and super critical extraction.
wherein the process of production of chlorinated sucrose comprises of,
i) deacylation of intermediates of chlorinated sucrose before as well as after drying of the chlorination reaction mixture by mild drying methods described above;
ii) use of alkali metal oxides as well as alkoxides, including Potassium Methoxide or Sodium Methoxide, for deacylation;
iii) achieving deacylation up to pH of 9 to 10.

2. The process of claim 1, wherein the chlorinated sucrose (or its intermediates or derivatives) containing liquid is a mixture of the respective substantially pure forms as well as of several solid ingredients of other chemicals in dissolved or suspended state.

3. The process of claim no. 2 wherein the individual ingredients of the said mixture of solids, containing chlorinated sucrose (or its intermediates or derivatives) as one of the ingredients, originate from reactants of a process undertaken for chlorination of sucrose-6-esters.

4. The process of claim no. 3 wherein the sucrose-6-ester is sucrose-6-acetate or sucrose-6-benzoate.

5. The process of claim no. 4 wherein the chlorinating reagent is any one suitable for chlorinating sucrose-6-ester.

6. The process of claim 5 wherein the said chlorinating reagent is a Vilsmeier reagent of the formula [XCIC = NR₂]⁺ Cl⁻ (where R represents an alkyl group and X represents a hydrogen atom or a methyl group].

7. The process of claim no. 3 wherein in the said process of chlorination, sequence of steps involves addition of sucrose-6-ester solution in a tertiary amide to the chlorinating reagent for chlorination.

8. The process of claim no. 7 wherein the said tertiary amide is N, N-dialkylformamide.

9. The process of claim no. 8 wherein the said N, N-dialkylformamide is dimethylformamide.

10. The process of claim 1, wherein the chlorinated sucrose containing liquid contains chlorinated sucrose in pure form with impurities in small or trace quantities.

11. The process of claim 10 wherein the said chlorinated sucrose containing liquid, is a wash solvent collected as effluent from a column chromatography of an impure solution of chlorinated sucrose.

12. The process of claim 11 wherein the said wash solvent is subjected to concentration before subjecting to drying treatment.

13. The process of claim 11 wherein the said wash solvent used for desorption is either a single solvent like ethyl acetate, or mixture of solvents like mixture of toluene and methanol or mixture of methanol or water & ethyl acetate.

14. The process of claim no. 11 when the said column chromatography is done by using a suitable adsorbent preferably, alumina or silica gel.

15. The process of claim 11 when the said impure solution is the crude extract of chlorinated sucrose (or its intermediates or derivatives) from a solid powder mixture of several chemicals, including chlorinated sucrose; extraction being done by any suitable extraction process including supercritical extraction or by conventional extraction in any suitable solvent including water, ethyl acetate, methanol, methyl ethyl ketone, acetone, which are capable of selective extraction of substantially pure form of chlorinated sucrose free from impurities.

16. The process of claim no 15 wherein the said solid powder mixture is the product of process of drying of reaction mixture as described in claim nos. 3 to 12.

17. The process of claim 12 wherein the concentrated extract is subjected to conventional crystallization for purification of chlorinated sugar.

18. The process of claim 3, wherein the said process of chlorination comprises of:
i) preparation of Vilsmeir reagent from Phosphorus oxy-chloride,
ii) addition of sucrose-6-ester, preferably sucrose-6-acetate, to Vilsmeier reagent at 5 to 10°C and allowing reaction to complete,
iii) heating the reaction mixture to 80 to 100 °C., preferably between 90 to 95 °C. and maintained for half to one hour,
iv) raising temperature of reaction mixture of step no. (iii) to 110 C., preferably to 120 to 130 °C. and maintained for 3-5 hours,
v) cooling the reaction mass to room temperature, cooling the reaction mass into a solution of a suitable deacylating reagent in inorganic basic solution like alkali hydroxide solution accompanied by further cooling to keep the temperature below 30 to 35 °C.,
vi) adjusting the pH to 7 to 9.5 and preferably 8-9.

19. The process of claim18 wherein at step no. v), wherein any alkoxide, preferably Potassium Methoxide or Sodium Methoxide is used instead of alkali metal hydroxides for deacylation.

20. The process of claim no. 18 wherein pH is adjusted only upto 9 and reaction mixture is subjected to drying as per claim 1.

21. The process of claim 1 wherein the solids obtained from drying of reaction mixture from chlorination step are extracted for chlorinated sucrose recovery by any suitable method of extraction, including, solvent extraction.

22. The process of claim 11 wherein the said impure solution is the solution of the solid powder mixture of several chemicals, including chlorinated sucrose, made in water and subjected to purification by application of separation methods including column chromatography, extraction in water immiscible solvent having selective affinity with chlorinated sucrose or chlorinated sucrose intermediates or chlorinated sucrose derivatives

23. The process of claim 11 when the said impure solution is the crude extract of chlorinated sucrose (or its intermediates or derivatives) from a solid powder mixture of several chemicals, including chlorinated sucrose; extraction being done by water and the water extract being subjected to a any suitable extraction process including to conventional extraction in any suitable solvent, including ethyl acetate, methanol, methyl ethyl ketone, acetone, which are capable of selective extraction of substantially pure form of chlorinated sucrose free from impurities.

24. A process according to any of claim 1 to claim 23, wherein at least part of the product is amorphous or non crystalline.

## Patentansprüche

1. Verfahren zum Handhaben einer Lösung aus Saccharose-Zwischenprodukten und Derivaten, die chlorierte Saccharose umfassen, wobei das Verfahren umfasst:
a) Entfernen von Flüssigkeiten aus der Lösung durch direktes Trocknen, unter Bedingungen, die mild genug sind, dass eine Verschlechterung oder Modifizierung der chlorierten Saccharose verhindert werden kann, zur Rückgewinnung von Feststoffen aus den Flüssigkeiten und wobei das Endprodukt von solchen Abläufen eine feste Masse aus den Chemikalien ist, die sichtlich frei von Flüssigkeit ist;
b) Zurückgewinnen der Feststoffe, die in der Flüssigkeit entweder in im Wesentlichen reiner Form oder mit anderen festen Verunreinigungen vorhanden sind;
c) wobei die Flüssigkeiten in einem Verfahrensschritt zum Herstellen einer chlorierten Saccharose, hauptsächlich 1',6'-Dichloro-1',6'-didesoxy-β-D-fructofuranosyl-4-chloro-4-desoxy-α-D-galactopyranosid, erhalten werden;
wobei der Verfahrensschritt des Trocknens ausgewählt ist aus Sprühtrocknung, Dünnschichttrocknung unter Rühren, Gefriertrocknung, und superkritische Extraktion davon oder eine Kombination umfasst,
wobei der Verfahrensschritt der Herstellung der chlorierten Saccharose umfasst
i) eine Deacylierung von Zwischenprodukten der chlorierten Saccharose vor sowie nach der Trocknung des Chlorierungsreaktionsgemischs durch milde Trocknungsverfahren, die vorstehend beschrieben sind;
ii) Verwendung von Alkalimetalloxiden sowie Alkoxiden, die Kaliummethoxid oder Natriummethoxid umfassen, zur Deacylierung;
iii) Erreichen einer Deacylierung bis zu einem pH von 9 bis 10.

2. Verfahren nach Anspruch 1, wobei die eine Flüssigkeit enthaltende chlorierte Saccharose (oder deren Zwischenprodukte oder Derivate) ein Gemisch aus den jeweiligen im Wesentlichen reinen Formen sowie den mehreren festen Inhaltsstoffen der anderen Chemikalien in einem gelösten oder suspendierten Zustand ist.

3. Verfahren nach Anspruch 2, wobei die einzelnen Inhaltsstoffe des Gemischs aus Feststoffen, die chlorierte Saccharose (oder deren Zwischenprodukte oder Derivate) als einen der Inhaltsstoffe enthalten, aus Ausgangsstoffen eines Verfahrens stammen, das zur Chlorierung von Saccharose-6-estern unternommen wurde.

4. Verfahren nach Anspruch 3, wobei der Saccharose-6-ester ein Saccharose-6-acetat oder ein Saccharose-6-benzoat ist.

5. Verfahren nach Anspruch 4, wobei das chlorierende Reagens ein beliebiges Reagens ist, das zum Chlorieren von Saccharose-6-ester geeignet ist.

6. Verfahren nach Anspruch 5, wobei es sich bei dem chlorierenden Reagens um ein Vilsmeier-Reagens der Formel [XC1C = NR₂]⁺Cl⁻ handelt (wobei R eine Alkylgruppe und X ein Wasserstoffatom oder eine Methylgruppe darstellt).

7. Verfahren nach Anspruch 3, wobei in dem Verfahren der Chlorierung, eine Abfolge von Schritten ein Hinzufügen einer Saccharose-6-ester-Lösung in einem tertiären Amid zu dem chlorierenden Reagens zur Chlorierung umfasst.

8. Verfahren nach Anspruch 7, wobei es sich bei dem tertiären Amid um N,N-Dialkylformamid handelt.

9. Verfahren nach Anspruch 8, wobei es sich bei dem N,N-Dialkylformamid um Dimethylformamid handelt.

10. Verfahren nach Anspruch 1, wobei die eine Flüssigkeit enthaltende chlorierte Saccharose chlorierte Saccharose in reiner Form mit in geringen Mengen oder Spuren enthaltenen Verunreinigungen enthält.

11. Verfahren nach Anspruch 10, wobei es sich bei der eine Flüssigkeit enthaltenden chlorierten Saccharose um ein Waschlösungsmittel handelt, das als ein Abfluss aus einer Säulenchromatographie von einer unreinen Lösung der chlorierten Saccharose erfasst wurde.

12. Verfahren nach Anspruch 11, wobei das Waschlösungsmittel vor einer Trocknungsbehandlung einer Konzentrationsbehandlung unterzogen wird.

13. Verfahren nach Anspruch 11, wobei das Waschlösungsmittel, das zur Desorption verwendet wird, entweder ein einzelnes Lösungsmittel wie Ethylacetat oder ein Gemisch aus Lösungsmitteln wie ein Gemisch aus Toluol und Methanol oder ein Gemisch aus Methanol oder Wasser und Ethylacetat ist.

14. Verfahren nach Anspruch 1, wobei die Säulenchromatographie durch Verwendung eines geeigneten Adsorbens, vorzugsweise Aluminiumoxid- oder Siliciumdioxidgel, ausgeführt wird.

15. Verfahren nach Anspruch 11, wobei die unreine Lösung das Rohextrakt der chlorierten Saccharose (deren Zwischenprodukten oder Derivaten) aus einem festen Pulvergemisch aus mehreren Chemikalien ist, die chlorierte Saccharose umfassen; wobei eine Extraktion durch einen geeigneten Extraktionsprozess ausgeführt wird, der eine superkritische Extraktion oder eine herkömmliche Extraktion in einem beliebigen geeigneten Lösungsmittel umfasst, das Wasser, Ethylacetat, Methanol, Methylethylketon, Aceton umfasst, die für eine selektive Extraktion von einer im Wesentlichen reinen Form von chlorierte Saccharose, die frei von Verunreinigungen ist, geeignet sind.

16. Verfahren nach Anspruch 15, wobei das feste Pulvergemisch das Produkt des Verfahrensschritts der Trocknung des Reaktionsgemisches ist, das in den Ansprüchen 3 bis 12 beschrieben ist.

17. Verfahren nach Anspruch 12, in dem das konzentrierte Extrakt einer herkömmlichen Kristallisierung zur Reinigung von chloriertem Zucker unterzogen wird.

18. Verfahren nach Anspruch 3, wobei das Verfahrensschritt der Chlorierung umfasst:
i) Erstellen eines Vilsmeier-Reagens aus Phosphoroxychlorid,
ii) Hinzufügen von Saccharose-6-ester, vorzugsweise Saccharose-6-acetat, zu dem Vilsmeier-Reagens bei 5 bis 10 °C und Ablaufenlassen der Reaktion,
iii) Erwärmen des Reaktionsgemischs auf 80 bis 100°C, vorzugsweise zwischen 90 und 95 °C, wobei die Temperatur eine halbe bis eine Stunde lang beibehalten wird,
iv) Erhöhen der Temperatur des Reaktionsgemisches von Schritt (iii) auf 110°C, vorzugsweise 120 bis 130 °C, wobei die Temperatur 3 bis 5 Stunden lang beibehalten wird,
v) Kühlen der Reaktionsmasse auf Raumtemperatur, Kühlen der Reaktionsmasse in einer Lösung aus einem geeigneten Deacylierungsreagens in einer anorganischen basischen Lösung wie einer Alkalihydroxidlösung, mit der ein weiteres Kühlen einhergeht, um die Temperatur unter 30 bis 35 °C zu halten.,
vi) Einstellen des pH von 7 auf 9,5 und vorzugsweise 8-9.

19. Verfahren nach Anspruch 18, wobei bei Schritt v), ein Alkoxid, vorzugsweise Kaliummethoxid oder Natriummethoxid, anstelle von Alkalimetalhydroxiden zur Deacylierung verwendet wird.

20. Verfahren nach Anspruch 18, wobei der pH nur bis auf 9 eingestellt wird und das Reaktionsgemisch einer Trocknung gemäß Anspruch 1 unterzogen wird.

21. Verfahren nach Anspruch 1, wobei die Feststoffe, die anhand des Trocknungsvorgangs des Reaktionsgemisches aus einem Chlorierungsschritt erhalten werden, zur Rückgewinnung von chlorierter Saccharose durch ein beliebiges geeignetes Verfahren der Extraktion, das eine Lösungsmittelextraktion umfasst, extrahiert werden.

22. Verfahren nach Anspruch 11, wobei es sich bei der unreinen Lösung um die Lösung des festen Pulvergemisches aus mehreren Chemikalien, die chlorierte Saccharose beinhalten, handelt, die in Wasser hergestellt und einer Reinigung durch Anwendung mehrerer Verfahren, die die Säulenchromatographie, Extraktion in einem mit Wasser unvermischbaren Lösungsmittel mit einer selektiven Affinität zu chlorierter Saccharose oder chlorierten Saccharose-Zwischenprodukten oder chlorierten Saccharose-Derivaten umfassen, unterzogen wurde.

23. Verfahren nach Anspruch 11, wobei die unreine Lösung das Rohextrakt der chlorierten Saccharose (oder deren Zwischenprodukten oder Derivaten) aus einem festen Pulvergemisch aus mehreren Chemikalien ist, die die chlorierte Saccharose umfassen; wobei die Extraktion durch Wasser erfolgt und das Wasserextrakt einem beliebigen geeigneten Extraktionsvorgang unterzogen wird, der eine herkömmliche Extraktion in einem beliebigen Lösungsmittel umfasst, das Ethylacetat, Methanol, Methylethylketon, Aceton umfasst, die für eine selektive Extraktion einer im Wesentlichen reinen Form von chlorierter Saccharose, die frei von Verunreinigungen ist, geeignet sind.

24. Verfahren nach einem der Ansprüche 1 bis 23, wobei zumindest ein Teil des Produktes amorph oder nicht-kristallin ist.

## Revendications

1. Procédé de manipulation d'une solution composée d'intermédiaires du saccharose et de dérivés de celui-ci, y compris le saccharose chloré, comprenant les étapes consistant à _{:}
a) éliminer les liquides de ladite solution par un séchage direct, dans des conditions assez douces pour empêcher la dégradation ou la modification du saccharose chloré, afin de récupérer les solides à partir desdits liquides et le produit final d'une telle opération est une masse solide de produits chimiques visiblement exempts dudit liquide ;
b) récupérer lesdits solides, présents dans ledit liquide qu'ils soient sous forme pratiquement pure ou comprenant d'autres impuretés solides ;
c) lesdits liquides étant obtenus par un procédé de production du saccharose chloré, principalement le 1',6'-dichloro-1', 6'-didésoxy-β-D-fructo-furanosyl-4-chloro-4-désoxy-α-D-galactopyranoside ;
ledit procédé de séchage comprenant un procédé ou une combinaison de procédés parmi ce qui suit : le séchage par agitation d'un film mince, le séchage par pulvérisation, la lyophilisation et l'extraction supercritique,
le procédé de production du saccharose chloré comprenant les étapes consistant à :
i) désacyler les intermédiaires du saccharose chloré avant ainsi qu'après séchage du mélange provenant de la réaction de chloration par des procédés de séchage doux tels que ceux décrits ci-dessus ;
ii) utiliser des oxydes de métaux alcalins ainsi que des alcoxydes, comprenant le méthoxyde de potassium ou le méthoxyde de sodium, pour la désacylation ;
iii) réaliser la désacylation jusqu'à un pH de 9 à 10.

2. Procédé selon la revendication 1, dans lequel le liquide contenant le saccharose chloré (ou ses intermédiaires ou dérivés) est un mélange des formes respectives pratiquement pures ainsi que de plusieurs ingrédients solides d'autres produits chimiques sous forme dissoute ou en suspension.

3. Procédé selon la revendication 2 dans lequel chacun des ingrédients dudit mélange de solides, contenant le saccharose chloré (ou ses intermédiaires ou dérivés) qui est l'un des ingrédients, provient des réactifs d'un procédé mis en ouvre pour la chloration de saccharose-6-esters.

4. Procédé selon la revendication 3 dans lequel le saccharose-6-ester est le saccharose-6-acétate ou le saccharose-6-benzoate.

5. Procédé selon la revendication 4 dans lequel le réactif de chloration est un réactif approprié quelconque pour chlorer le saccharose-6-ester.

6. Procédé selon la revendication 5 dans lequel ledit réactif de chloration est un réactif de Vilsmeier de formule [XClC = NR₂]⁺ Cl⁻ (dans laquelle R représente un groupe alkyle et X représente un atome d'hydrogène ou un groupe méthyle).

7. Procédé selon la revendication 3 dans lequel, dans ledit procédé de chloration, la séquence d'étapes comprend l'addition d'une solution de saccharose-6-ester dans un amide tertiaire au réactif de chloration pour la chloration.

8. Procédé selon la revendication 7 dans lequel ledit amide tertiaire est le N,N-dialkylformamide.

9. Procédé selon la revendication 8 dans lequel ledit N,N-dialkylformamide est le diméthylformamide.

10. Procédé selon la revendication 1, dans lequel le liquide contenant le saccharose chloré contient du saccharose chloré sous forme pure avec des impuretés en petites quantités ou à l'état de traces.

11. Procédé selon la revendication 10 dans lequel ledit liquide contenant le saccharose chloré est un solvant de lavage collecté comme effluent d'une chromatographie colonne d'une solution impure de saccharose chloré.

12. Procédé selon la revendication 11 dans lequel ledit solvant de lavage est concentré avant d'être soumis à un traitement par séchage.

13. Procédé selon la revendication 11 dans lequel ledit solvant de lavage utilisé pour la désorption est soit un solvant unique comme de l'acétate d'éthyle, ou un mélange de solvants comme un mélange de toluène et de méthanol ou un mélange de méthanol ou d'eau et d'acétate d'éthyle.

14. Procédé selon la revendication 11 dans lequel ladite chromatographie colonne est réalisée en utilisant un adsorbant approprié, de préférence de l'alumine ou du gel de silice.

15. Procédé selon la revendication 11 dans lequel ladite solution impure est l'extrait brut de saccharose chloré (ou de ses intermédiaires ou dérivés) provenant d'un mélange de poudres solides de plusieurs produits chimiques, y compris le saccharose chloré ; l'extraction étant réalisée par un quelconque procédé d'extraction y compris l'extraction supercritique ou par une extraction conventionnelle dans un quelconque solvant approprié y compris l'eau, l'acétate d'éthyle, le méthanol, la méthyl éthyl cétone, l'acétone, qui sont capables de réaliser une extraction sélective d'une forme pratiquement pure du saccharose chloré exempte d'impuretés.

16. Procédé selon la revendication 15 dans lequel ledit mélange de poudres solides est le produit du procédé de séchage du mélange réactionnel tel que décrit dans les revendications 3 à 12.

17. Procédé selon la revendication 12 dans lequel l'extrait concentré est soumis à une cristallisation conventionnelle afin de purifier le sucre chloré.

18. Procédé selon la revendication 3, dans lequel ledit procédé de chloration comprend les étapes consistant à :
i) préparer un réactif de Vilsmeier à partir d'oxychlorure de phosphore,
ii) ajouter le saccharose-6-ester, de préférence le saccharose-6-acétate, dans le réactif de Vilsmeier à une température de 5 à 10°C et laisser la réaction se terminer,
iii) chauffer le mélange réactionnel à une température de 80 à 100 °C, de préférence de 90 à 95 °C, et maintenue pendant une demi-heure à une heure,
iv) augmenter la température du mélange réactionnel de l'étape (iii) à 110°C, de préférence à une température de 120 à 130 °C, et maintenue pendant 3 à 5 heures,
v) refroidir la masse réactionnelle jusqu'à température ambiante, refroidir la masse réactionnelle à l'intérieur d'une solution d'un réactif désacylant approprié dans une solution basique inorganique comme une solution d'hydroxyde alcalin, cette étape étant accompagnée d'une étape consistant à refroidir de nouveau la solution afin de garder la température inférieure à une température de 30 à 35 °C,
vi) ajuster le pH à une valeur de 7 à 9,5 et de préférence de 8 à 9.

19. Procédé selon la revendication 18 dans lequel, à l'étape v), un alcoxyde quelconque, de préférence le méthoxyde de potassium ou le méthoxyde de sodium est utilisé au lieu d'un hydroxyde de métal alcalin pour la désacylation.

20. Procédé selon la revendication 18 dans lequel le pH n'est ajusté qu'à une valeur allant jusqu'à 9 et le mélange réactionnel est soumis à un séchage comme pour la revendication 1.

21. Procédé selon la revendication 1 dans lequel les solides obtenus à partir du séchage du milieu réactionnel provenant de l'étape de chloration sont extraits afin de récupérer le saccharose chloré par un procédé d'extraction approprié quelconque, y compris une extraction par un solvant.

22. Procédé selon la revendication 11 dans lequel ladite solution impure est la solution du mélange de poudres solides de plusieurs produits chimiques, y compris le saccharose chloré, dans de l'eau et soumise à une purification par l'application de procédés de séparation comprenant la chromatographie colonne, l'extraction dans un solvant non miscible à l'eau ayant une affinité sélective avec le saccharose chloré ou des intermédiaires du saccharose chloré ou des dérivés du saccharose chloré.

23. Procédé selon la revendication 11 dans lequel ladite solution impure est l'extrait brut de saccharose chloré (ou de ses intermédiaires ou dérivés) provenant d'un mélange de poudres solides de plusieurs produits chimiques, y compris le saccharose chloré ; l'extraction étant réalisée par de l'eau et l'extrait aqueux étant soumis à un quelconque procédé d'extraction approprié comprenant une extraction conventionnelle dans un quelconque solvant approprié, y compris l'acétate d'éthyle, le méthanol, la méthyl éthyl cétone, l'acétone, qui sont capables de réaliser une extraction sélective d'une forme pratiquement pure du saccharose chloré exempte d'impuretés.

24. Procédé selon l'une quelconque des revendications 1 à 23, dans lequel au moins une partie du produit est amorphe ou n'est pas cristalline.
